# EUROPEAN PATENT APPLICATION

(11) **EP 4 502 600 A1**
(43) Date of publication of application: **05.02.2025**
(21) Application number: 23778812.0
(22) Date of filing: 30.01.2023
(51) Int. Cl.: G01N 33/48, G01N 21/17, G01N 33/483, G06T 7/00, G06T 7/62, G06V 10/70, G06V 10/74, G06V 20/69

(54) **DRUG DEVELOPMENT SUPPORT DEVICE, METHOD FOR OPERATING DRUG DEVELOPMENT SUPPORT DEVICE, AND PROGRAM FOR OPERATING DRUG DEVELOPMENT SUPPORT DEVICE**

(30) Priority: 29.03.2022 JP 2022054509
(71) Applicant: FUJIFILM Corporation, Tokyo 106-8620 (JP)
(72) Inventor: TOMINAGA, Shunsuke, Ashigarakami-gun, Kanagawa 258-8577 (JP)
(74) Representative: HGF
(86) International application number: PCT/JP2023/002930
(87) International publication number: WO 2023/188775

(57) **Abstract**

A drug discovery support apparatus includes a processor configured to: acquire a first section image obtained by imaging a tissue section of at least one of a plurality of subjects constituting an administration group to which a candidate substance for a drug has been administered; extract a first feature amount from the first section image; acquire a plurality of second section images obtained by imaging tissue sections of a plurality of subjects that have the same attributes and are placed under the same breeding environment as the subjects constituting the administration group and that constitute a control group to which the candidate substance for a drug has not been administered; extract a second feature amount from each of the plurality of second section images; acquire third feature amount information related to a third feature amount extracted from each of a plurality of third section images obtained by imaging tissue sections of a plurality of subjects constituting a control group to which the candidate substance was not administered in a past evaluation test; and determine whether or not an abnormality is present in the tissue section included in the first section image on the basis of the first feature amount, the second feature amount, and the third feature amount information.

## Description

### BACKGROUND OF THE INVENTION

### 1. Field of the Invention

The technology of the present disclosure relates to a drug discovery support apparatus, a method for operating a drug discovery support apparatus, and a program for operating a drug discovery support apparatus.

### 2. Description of the Related Art

In a field of drug discovery, a test is performed which administers a candidate substance for a drug to a subject, such as a rat, and evaluates the efficacy and toxicity of the candidate substance. In this evaluation test, a section image obtained by imaging a tissue section (for example, a brain section, a liver section, or a heart section) collected by autopsying the subject is used. In the related art, a pathologist observes the section image to perform the evaluation. However, with the recent progress of an image analysis technique, a technique has been developed that automatically performs the evaluation without causing the pathologist trouble.

For example, JP2021-508373A discloses a technique that performs evaluation with a machine learning model trained using a section image obtained by imaging a tissue section (referred to as a "tissue sample without a substantial abnormality" in JP2021-508373A) in which an abnormality, such as a tumor or inflammation, has not occurred. Specifically, in JP2021-508373A, first, a section image of a subject, to which a candidate substance has been administered, is input to the machine learning model. Then, a difference (referred to as "deviation from a normal score" in JP2021-508373A) between a feature amount extracted from the section image and a feature amount extracted from a section image for learning obtained by imaging a tissue section, in which no abnormality has occurred, is calculated. Then, it is determined whether or not an abnormality occurs in the tissue section of the subject, to which the candidate substance has been administered, on the basis of the calculated difference.

### SUMMARY OF THE INVENTION

The subjects are divided into an administration group to which the candidate substance is administered and a control group to which the candidate substance is not administered for comparison with the administration group. Abnormalities may occur in some subjects in the control group even though the candidate substance is not administered. It is considered that the abnormalities occur in the subjects in the control group due to an attribute, a weekly age, and/or a breeding environment, such as feed. The subject in which an abnormality occurs due to the attribute and/or the breeding environment is also present in the administration group. Therefore, in a case where an abnormality occurs in the tissue section of the subject in the administration group, it is necessary to determine whether or not the cause of the abnormality is really the candidate substance. However, JP2021-508373A does not disclose the abnormality that occurs due to the attribute and/or the breeding environment and, of cause, does not disclose a method for determining whether or not the cause of the abnormality is the candidate substance.

An embodiment according to the technology of the present disclosure provides a drug discovery support apparatus, a method for operating a drug discovery support apparatus, and a program for operating a drug discovery support apparatus that can determine whether or not an abnormality caused by a candidate substance for a drug has occurred in a subject in an administration group.

According to an aspect of the present disclosure, there is provided a drug discovery support apparatus comprising a processor configured to: acquire a first section image obtained by imaging a tissue section of at least one of a plurality of subjects constituting an administration group to which a candidate substance for a drug has been administered; extract a first feature amount from the first section image; acquire a plurality of second section images including tissue sections of a plurality of subjects that have the same attributes and are placed under the same breeding environment as the subjects constituting the administration group and that constitute a control group to which the candidate substance for a drug has not been administered; extract a second feature amount from each of the plurality of second section images; acquire third feature amount information related to a third feature amount extracted from each of a plurality of third section images including tissue sections of a plurality of subjects constituting a control group to which the candidate substance was not administered in a past evaluation test; and determine whether or not an abnormality is present in the tissue section included in the first section image on the basis of the first feature amount, the second feature amount, and the third feature amount information.

Preferably, the processor is configured to extract the first feature amount and the second feature amount using a machine learning model trained using the plurality of third section images.

Preferably, the first feature amount, the second feature amount, and the third feature amount have the same number of dimensions and are comparable in the same feature amount space, and the third feature amount information is information of a representative position of the plurality of third feature amounts in the feature amount space. Preferably, the processor is configured to: calculate a first distance between the representative position and a position of the first feature amount in the feature amount space; calculate a second distance between the representative position and a position of each of the plurality of second feature amounts in the feature amount space; and perform the determination on the basis of the first distance and the second distance.

Preferably, the processor is configured to: extract the first feature amount for each of a plurality of first patch images obtained by subdividing the first section image and calculate the first distance for each of the plurality of first patch images; extract the second feature amount for each of a plurality of second patch images obtained by subdividing the second section image and calculate the second distance for each of the plurality of second patch images; and perform the determination on the basis of a first area ratio of the first patch images, for which the first distance is out of a setting range, to the first section image and a second area ratio of the second patch images, for which the second distance is out of the setting range, to the second section image.

Preferably, the processor is configured to determine that the abnormality is present in the tissue section included in the first section image in a case where there is a statistically significant difference between the first area ratio and the second area ratio.

Preferably, the processor is configured to determine that the abnormality is present in the tissue section included in the first section image for which the first area ratio is greater than a threshold value set on the basis of the second area ratio.

Preferably, the first feature amount, the second feature amount, and the third feature amount have the same number of dimensions and are comparable in the same feature amount space, and the third feature amount information is information of a representative position of the plurality of third feature amounts in the feature amount space. Preferably, the processor is configured to: calculate a first distance between the representative position and a position of the first feature amount in the feature amount space; calculate a third distance between a representative position of a plurality of the second feature amounts and the position of the first feature amount in the feature amount space; and perform the determination on the basis of the first distance and the third distance.

Preferably, the processor is configured to determine that the abnormality is present in the tissue section included in the first section image in a case where a weighted sum of the first distance and the third distance is out of a setting range.

Preferably, the processor is configured to receive an instruction to set weighting coefficients, by which the first distance and the third distance are multiplied, in the weighted sum.

Preferably, the first section image and the second section image are images obtained by imaging a slide specimen on which tissue sections of a plurality of types of organs are placed, and the processor is configured to: identify the tissue sections of each organ from the first section image and the second section image; extract the first feature amount and the second feature amount of each of the identified tissue sections of each organ; and determine whether or not an abnormality is present in each of the tissue sections of each organ included in the first section image on the basis of the first feature amount, the second feature amount, and the third feature amount information.

According to another aspect of the present disclosure, there is provided a method for operating a drug discovery support apparatus. The method comprises: acquiring a first section image obtained by imaging a tissue section of at least one of a plurality of subjects constituting an administration group to which a candidate substance for a drug has been administered; extracting a first feature amount from the first section image; acquiring a plurality of second section images including tissue sections of a plurality of subjects that have the same attributes and are placed under the same breeding environment as the subjects constituting the administration group and that constitute a control group to which the candidate substance for a drug has not been administered; extracting a second feature amount from each of the plurality of second section images; acquiring third feature amount information related to a third feature amount extracted from each of a plurality of third section images including tissue sections of a plurality of subjects constituting a control group to which the candidate substance was not administered in a past evaluation test; and determining whether or not an abnormality is present in the tissue section included in the first section image on the basis of the first feature amount, the second feature amount, and the third feature amount information.

According to still another aspect of the present disclosure, there is provided a program for operating a drug discovery support apparatus. The program causes a computer to execute a process comprising: acquiring a first section image obtained by imaging a tissue section of at least one of a plurality of subjects constituting an administration group to which a candidate substance for a drug has been administered; extracting a first feature amount from the first section image; acquiring a plurality of second section images obtained by imaging tissue sections of a plurality of subjects that have the same attributes and are placed under the same breeding environment as the subjects constituting the administration group and that constitute a control group to which the candidate substance for a drug has not been administered; extracting a second feature amount from each of the plurality of second section images; acquiring third feature amount information related to a third feature amount extracted from each of a plurality of third section images obtained by imaging tissue sections of a plurality of subjects constituting a control group to which the candidate substance was not administered in a past evaluation test; and determining whether or not an abnormality is present in the tissue section included in the first section image on the basis of the first feature amount, the second feature amount, and the third feature amount information.

According to the technology of the present disclosure, it is possible to provide a drug discovery support apparatus, a method for operating a drug discovery support apparatus, and a program for operating a drug discovery support apparatus that can determine whether or not an abnormality caused by a candidate substance for a drug has occurred in a subject in an administration group.

### BRIEF DESCRIPTION OF THE DRAWINGS

Fig. 1 is a diagram illustrating steps of an evaluation test, section images, and a drug discovery support apparatus.
Fig. 2 is a diagram illustrating a current administration group and a current control group.
Fig. 3 is a block diagram illustrating a computer constituting the drug discovery support apparatus.
Fig. 4 is a block diagram illustrating processing units of a CPU of the drug discovery support apparatus.
Fig. 5 is a diagram illustrating first patch images obtained by subdividing a first section image.
Fig. 6 is a diagram illustrating an aspect in which a first feature amount is extracted from the first patch image by a feature amount extractor.
Fig. 7 is a diagram illustrating an aspect in which a second feature amount is extracted from a second patch image by the feature amount extractor.
Fig. 8 is a diagram illustrating a structure of the feature amount extractor.
Fig. 9 is a diagram illustrating a process in a learning phase of an autoencoder.
Fig. 10 is a diagram illustrating a past control group.
Fig. 11 is a diagram illustrating an aspect in which a third feature amount is extracted from a third patch image by the feature amount extractor.
Fig. 12 is a graph illustrating the third feature amounts plotted in a feature amount space and a diagram illustrating third feature amount information.
Fig. 13 is a diagram illustrating a first distance and a second distance.
Fig. 14 is a diagram illustrating an aspect in which a determination unit outputs a comparison result between the first distance and a setting range.
Fig. 15 is a diagram illustrating an aspect in which the determination unit outputs a comparison result between a second distance and the setting range.
Fig. 16 is a diagram illustrating a first area ratio.
Fig. 17 is a diagram illustrating an aspect in which a plurality of second area ratios are calculated from a plurality of second section images.
Fig. 18A is a diagram illustrating a process of the determination unit in a case where a p-value in a statistical hypothesis test using the first area ratio and the second area ratio is equal to or greater than a significance level, and Fig. 18B is a diagram illustrating a process of the determination unit in a case where the p-value is less than the significance level.
Fig. 19 is a diagram illustrating a drug discovery support screen before an abnormality determination instruction is input.
Fig. 20 is a diagram illustrating a drug discovery support screen after the abnormality determination instruction is input.
Fig. 21 is a flowchart illustrating a processing procedure of the drug discovery support apparatus.
Fig. 22 is a diagram illustrating another example of the process of the determination unit.
Fig. 23 is a diagram illustrating a process of a determination unit according to a 2_1-th embodiment.
Fig. 24 is a diagram illustrating a 2_2-th embodiment in which a coefficient setting instruction is received.
Fig. 25 is a diagram illustrating a third embodiment in which a section image obtained by imaging a slide specimen on which tissue sections of a plurality of types of organs are placed is handled.
Fig. 26 is a diagram illustrating an aspect in which a first feature amount is extracted from a first patch image obtained by subdividing a heart section by a feature amount extractor for a heart section.
Fig. 27 is a diagram illustrating another example of the drug discovery support screen.

### DESCRIPTION OF THE PREFERRED EMBODIMENTS

### [First Embodiment]

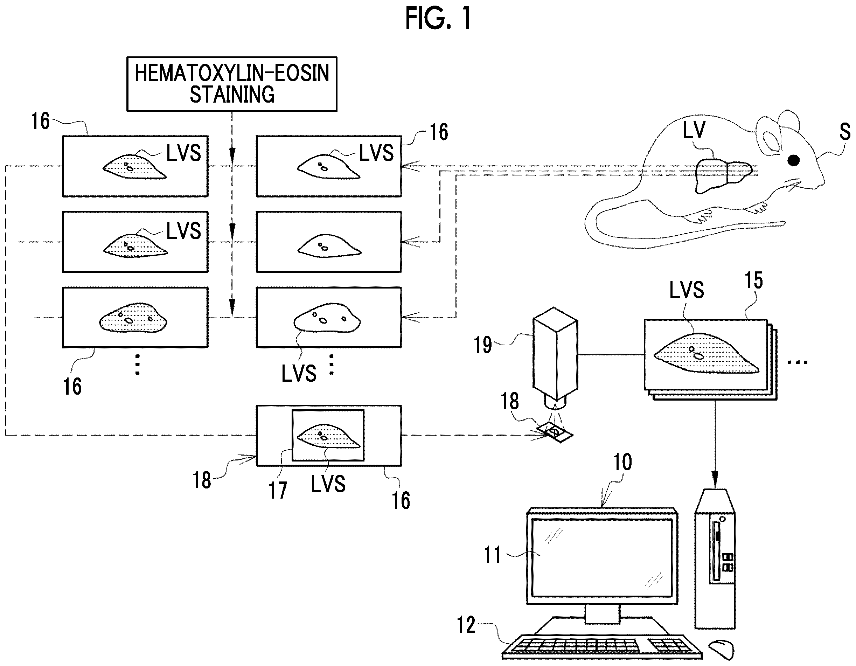

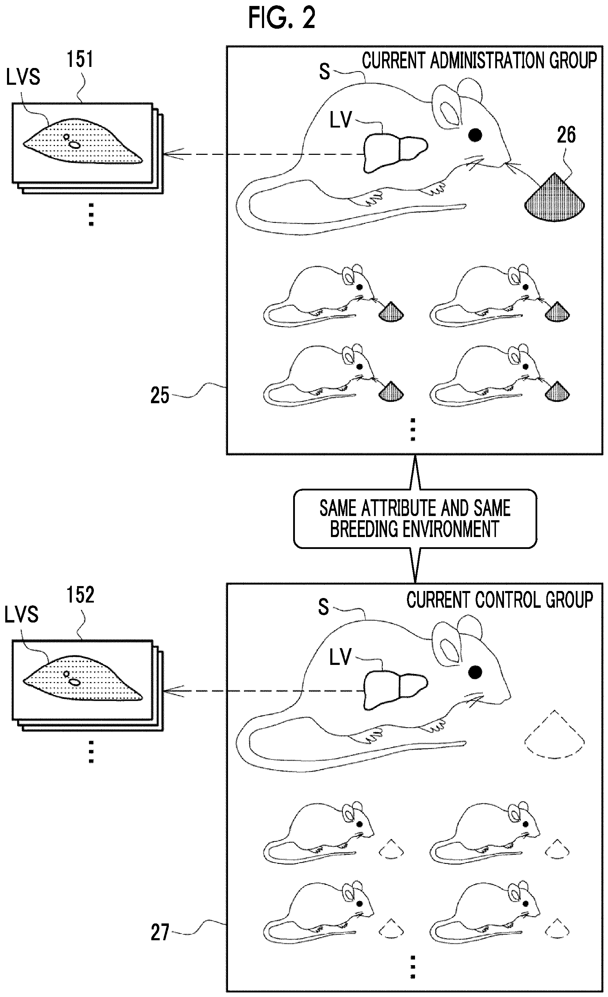

For example, as illustrated in Fig. 1, a drug discovery support apparatus 10 according to the present disclosure is used to evaluate the efficacy and toxicity of a candidate substance 26 for a drug (see Fig. 2). The drug discovery support apparatus 10 is, for example, a desktop personal computer and comprises a display 11 that displays various screens and an input device 12 that consists of a keyboard, a mouse, a touch panel, and/or a microphone for voice input. The drug discovery support apparatus 10 is operated by an operator such as a staff member of a pharmaceutical facility involved in drug development.

A section image 15 is input to the drug discovery support apparatus 10. The section image 15 is generated, for example, by the following procedure. First, a subject S, such as a rat, that is prepared to evaluate the candidate substance 26 is autopsied, and a plurality of tissue sections (hereinafter, referred to as liver sections) LVS of cross sections of an organ, here, a liver LV of the subject S are collected. Then, the collected liver sections LVS are attached one by one to a slide glass 16. Then, the liver sections LVS are stained, here, with a hematoxylin-eosin dye. Then, the stained liver sections LVS are covered with a cover glass 17 to complete a slide specimen 18. Then, the slide specimen 18 is set in an imaging device 19, such as a digital optical microscope, and the section image 15 is captured by the imaging device 19. The section image 15 obtained in this way is given subject identification data (ID) for uniquely identifying the subject S, an image ID for uniquely identifying the section image 15, an imaging date and time, and the like. In addition, the staining may be staining with a hematoxylin dye alone, staining with a nuclear fast red dye, or the like.

For example, as illustrated in Fig. 2, the section images 15 input to the drug discovery support apparatus 10 include a first section image 151 and a second section image 152. The first section image 151 is an image obtained by imaging the liver section LVS of the subject S in a current administration group 25. The current administration group 25 is composed of a plurality of subjects S to which the candidate substance 26 for a drug has been administered. The number of subjects S constituting the current administration group 25 is, for example, about 5 to 10. Since a plurality of first section images 151 are obtained from one subject S, the number of first section images 151 obtained from the current administration group 25 is a value obtained by multiplying the number of first section images 151 obtained from one subject S by the number of subjects S. For example, in a case where the number of first section images 151 obtained from one subject S is 10 and the number of subjects S constituting the current administration group 25 is 10, 100 (= 10 × 10) first section images 151 are obtained.

The second section image 152 is an image obtained by imaging the liver section LVS of the subject S in a current control group 27. The current control group 27 is composed of a plurality of subjects S to which the candidate substance 26 for a drug has not been administered, contrary to the current administration group 25. The subjects S constituting the current control group 27 have the same attributes and are placed under the same breeding environment as the subjects S constituting the current administration group 25. The same attributes include, for example, the same weekly age and/or the same gender. In addition, the same attributes also include the same weekly age composition ratio and/or the same gender composition ratio (for example, five males and five females). The same breeding environment means, for example, that feed is the same, that the temperature and humidity of a breeding space are the same, and/or that the size of the breeding space is the same. The "same" in the same breeding environment indicates not only the exact same, but also the same including an error that is generally allowed in the technical field to which the technology of the present disclosure belongs and that does not go against the gist of the technology of the present disclosure. The number of subjects S constituting the current control group 27 is also, for example, about 5 to 10 that is equal to the number of subjects S in the current administration group 25. Similarly to the first section images 151, a plurality of second section images 152 are obtained from one subject S. Therefore, the number of second section images 152 obtained from the current control group 27 is a value obtained by multiplying the number of second section images 152 obtained from one subject S by the number of subjects S.

Further, a plurality of groups having different doses of the candidate substance 26 are present in the current administration group 25. For example, the dose of the candidate substance 26 varies in three stages of a high-dose group, a medium-dose group, and a low-dose group. This makes it possible to determine the influence of the dose of the candidate substance 26 on the subject S.

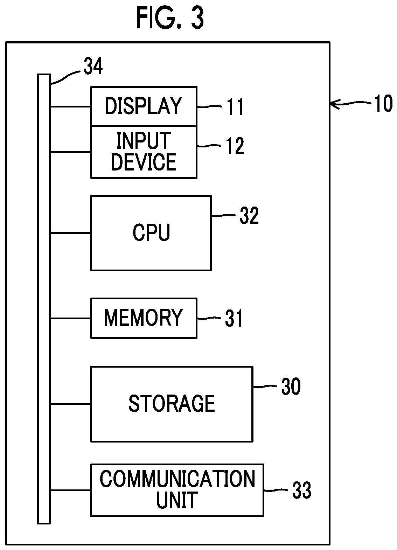

For example, as illustrated in Fig. 3, a computer constituting the drug discovery support apparatus 10 comprises a storage 30, a memory 31, a central processing unit (CPU) 32, and a communication unit 33 in addition to the display 11 and the input device 12. These components are connected to one another via a bus line 34.

The storage 30 is a hard disk drive that is provided in the computer constituting the drug discovery support apparatus 10 or that is connected via a cable or a network. Alternatively, the storage 30 is a disk array in which a plurality of hard disk drives are connected in series. The storage 30 stores a control program, such as an operating system, various application programs, various types of data associated with these programs, and the like. In addition, a solid state drive may be used instead of the hard disk drive.

The memory 31 is a work memory for the CPU 32 to execute processes. The CPU 32 loads the program stored in the storage 30 into the memory 31 and executes a process corresponding to the program. Therefore, the CPU 32 controls the overall operation of each unit of the computer. In addition, the CPU 32 is an example of a "processor" according to the technology of the present disclosure. Further, the memory 31 may be provided in the CPU 32. The communication unit 33 controls the transmission of various types of information to an external device such as the imaging device 19.

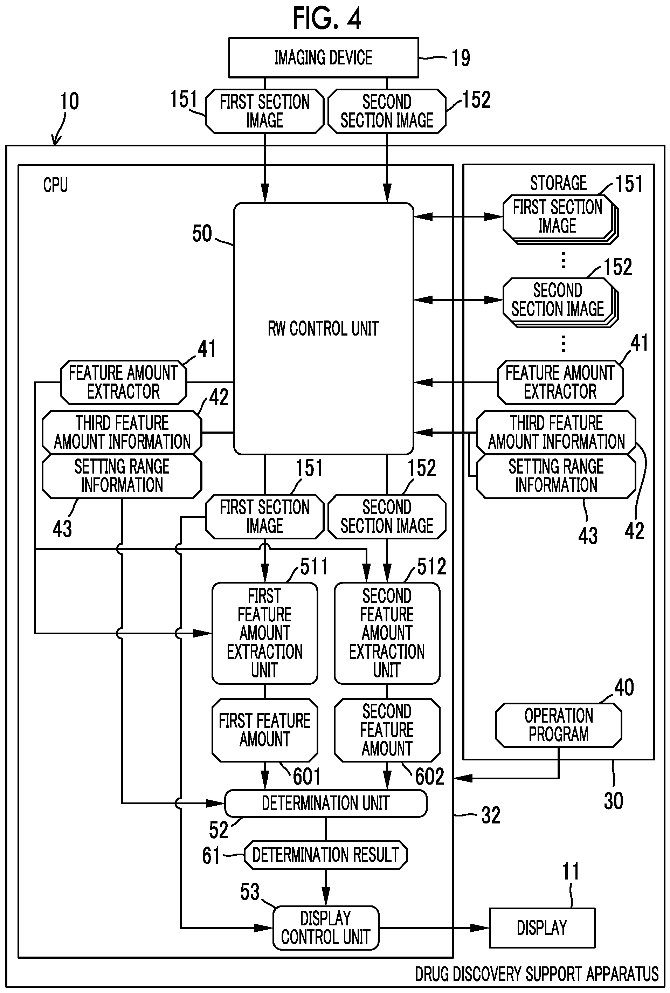

For example, as illustrated in Fig. 4, an operation program 40 is stored in the storage 30 of the drug discovery support apparatus 10. The operation program 40 is an application program for causing the computer to function as the drug discovery support apparatus 10. That is, the operation program 40 is an example of a "program for operating a drug discovery support apparatus" according to the technology of the present disclosure. A feature amount extractor 41, third feature amount information 42, setting range information 43, and the like are also stored in the storage 30. The feature amount extractor 41 is an example of a "machine learning model" according to the technology of the present disclosure.

In a case where the operation program 40 is started, the CPU 32 of the computer constituting the drug discovery support apparatus 10 functions as a read write (hereinafter, abbreviated to RW) control unit 50, a first feature amount extraction unit 511, a second feature amount extraction unit 512, a determination unit 52, and a display control unit 53 in cooperation with the memory 31 and the like.

The RW control unit 50 controls the storage of various types of data in the storage 30 and the reading-out of various types of data in the storage 30. For example, the RW control unit 50 stores the first section image 151 and the second section image 152 from the imaging device 19 in the storage 30.

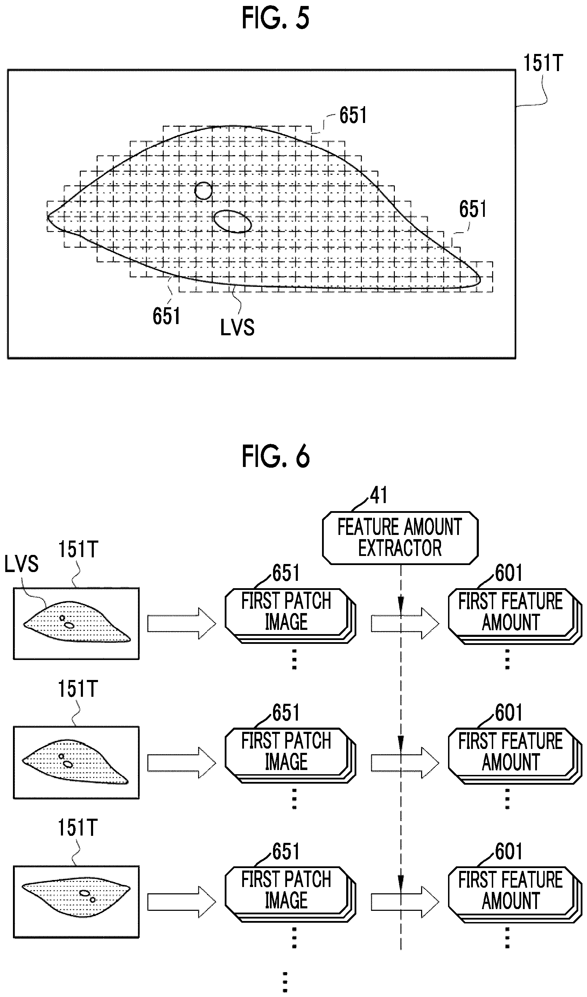

The RW control unit 50 reads out the first section image 151 from the storage 30 to acquire the first section image 151. The RW control unit 50 outputs the first section image 151 to the first feature amount extraction unit 511 and the display control unit 53. The first section image 151 output from the RW control unit 50 to the first feature amount extraction unit 511 is an object in which whether or not an abnormality is present in the liver section LVS is to be determined. Hereinafter, the first section image 151 in which whether or not an abnormality is present in the liver section LVS is to be determined is referred to as a target first section image 151T (see Fig. 5 and the like).

The RW control unit 50 reads out a plurality of second section images 152 from the storage 30 to acquire the plurality of second section images 152. The RW control unit 50 outputs all of the second section images 152 to the second feature amount extraction unit 512.

The RW control unit 50 reads out the feature amount extractor 41 from the storage 30 and outputs the feature amount extractor 41 to the first feature amount extraction unit 511 and the second feature amount extraction unit 512. In addition, the RW control unit 50 reads out the third feature amount information 42 from the storage 30 to acquire the third feature amount information 42 and outputs the third feature amount information 42 to the determination unit 52. Further, the RW control unit 50 reads out the setting range information 43 from the storage 30 and outputs the setting range information 43 to the determination unit 52.

The first feature amount extraction unit 511 extracts a first feature amount 601 from the target first section image 151T using the feature amount extractor 41. The first feature amount extraction unit 511 outputs the first feature amount 601 to the determination unit 52.

The second feature amount extraction unit 512 extracts a second feature amount 602 from each of the second section images 152 using the feature amount extractor 41. The second feature amount extraction unit 512 outputs the second feature amount 602 to the determination unit 52.

The determination unit 52 determines whether or not an abnormality caused by the candidate substance 26 is present in the liver section LVS included in the target first section image 151T on the basis of the first feature amount 601, the second feature amount 602, the third feature amount information 42, and the setting range information 43. Examples of the abnormality include lesions, such as tumors, inflammation, cysts, and infiltrates, that are not seen in the normal liver section LVS. The determination unit 52 outputs, to the display control unit 53, a determination result 61 of whether or not the abnormality caused by the candidate substance 26 is present in the liver section LVS included in the target first section image 151T.

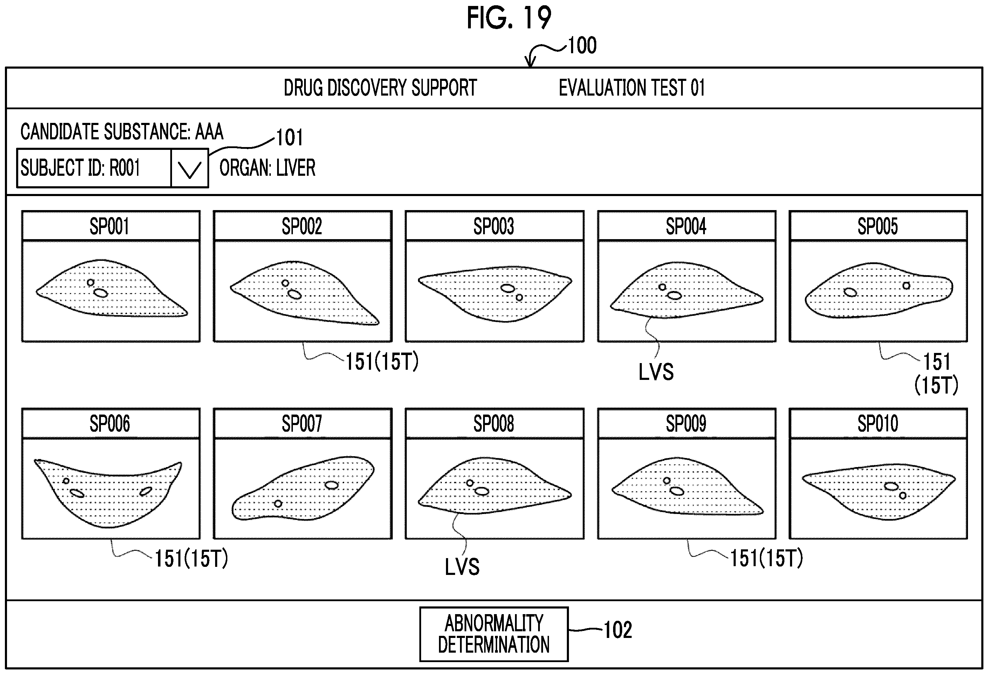

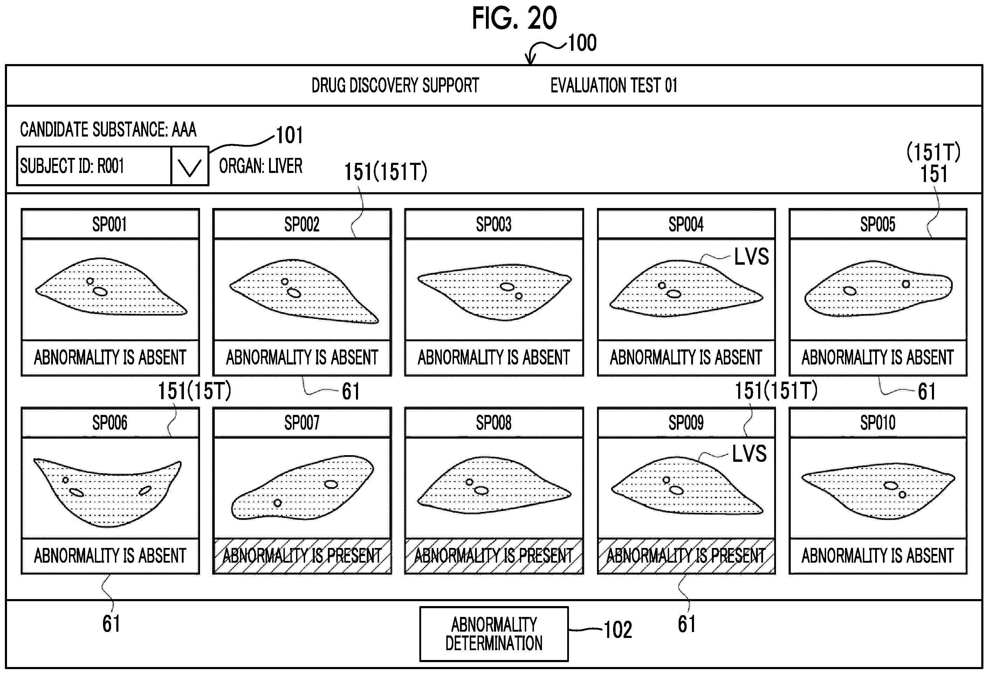

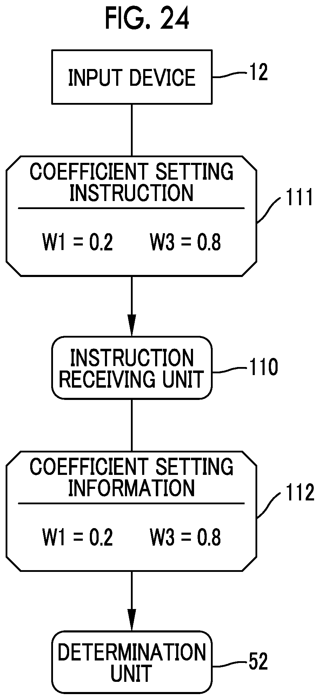

The display control unit 53 performs control to display various screens on the display 11. The various screens include, for example, a drug discovery support screen 100 (see Figs. 19 and 20) on which the first section image 151 and the determination result 61 are displayed. Further, in addition to the processing units 50 to 53, an instruction receiving unit 110 (see Fig. 24) that receives various operation instructions from the input device 12 and the like are constructed in the CPU 32.

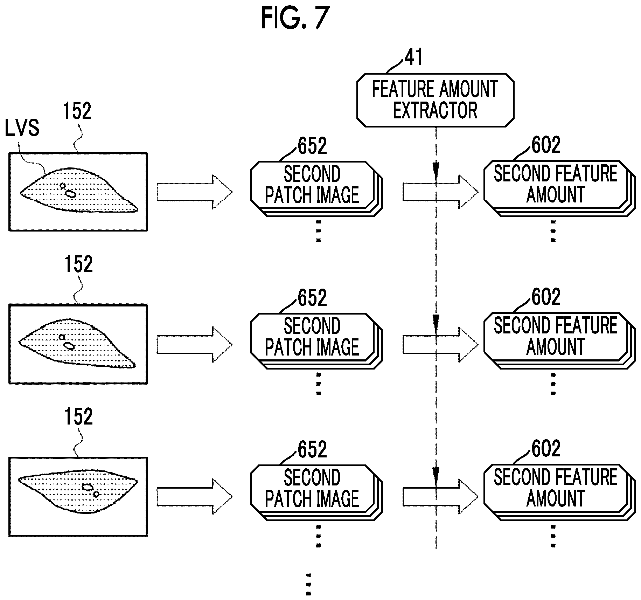

For example, as illustrated in Fig. 5, the first feature amount extraction unit 511 recognizes the liver section LVS included in the target first section image 151T using a well-known image recognition technique and subdivides the recognized liver section LVS into a plurality of first patch images 651. The first patch image 651 has a preset size that can be handled by the feature amount extractor 41. Similarly, the second feature amount extraction unit 512 subdivides the liver section LVS included in the second section image 152 into a plurality of second patch images 652 (see Fig. 7), which is not illustrated. Further, in Fig. 5, the first patch image 651 does not have a region that overlaps other first patch images 651. However, the first patch image 651 may partially overlap other first patch images 651. The same applies to the second patch images 652.

For example, as illustrated in Fig. 6, the first feature amount extraction unit 511 extracts the first feature amount 601 for each of a plurality of first patch images 651 obtained by subdividing the target first section image 151T, using the feature amount extractor 41. In addition, for example, as illustrated in Fig. 7, the second feature amount extraction unit 512 extracts the second feature amount 602 for each of a plurality of second patch images 652 obtained by subdividing all of the second section images 152, using the feature amount extractor 41.

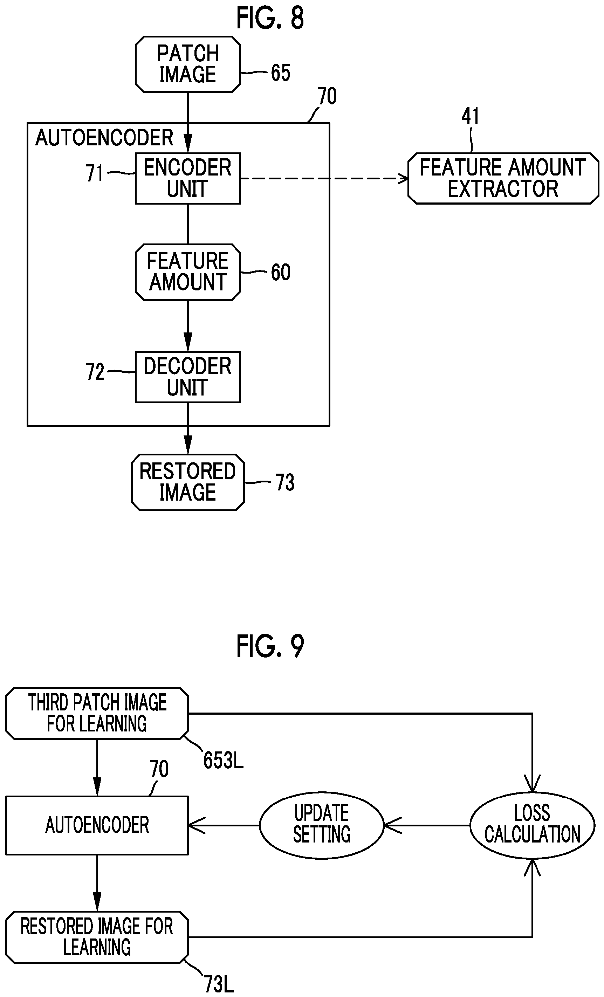

For example, as illustrated in Fig. 8, an encoder unit 71 of an autoencoder 70 is used as the feature amount extractor 41. The autoencoder 70 includes a decoder unit 72 in addition to the encoder unit 71. Patch images 65, such as the first patch image 651 and the second patch image 652, are input to the encoder unit 71. The encoder unit 71 converts the patch image 65 into a feature amount 60. The encoder unit 71 transmits the feature amount 60 to the decoder unit 72. The decoder unit 72 generates a restored image 73 of the patch image 65 from the feature amount 60.

As is well known, the encoder unit 71 includes a convolutional layer that performs a convolution process using a filter, a pooling layer that performs a pooling process, such as a maximum pooling process, and the like. The same applies to the decoder unit 72. The encoder unit 71 repeatedly performs the convolution process using the convolutional layer and the pooling process using the pooling layer on the input patch image 65 a plurality of times to extract the feature amount 60. The extracted feature amount 60 indicates the shape and texture features of the liver section LVS included in the patch image 65.

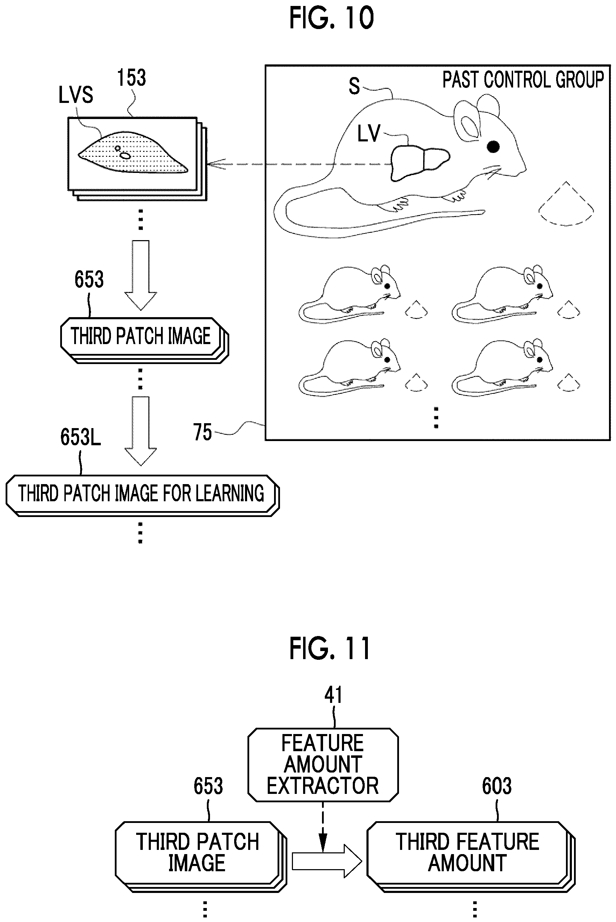

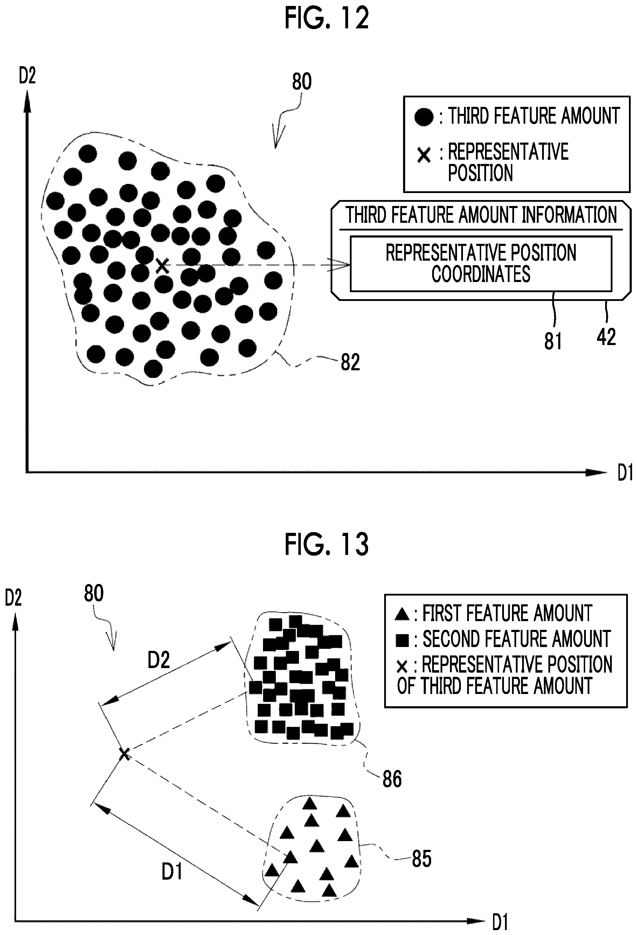

The feature amount 60 is a set of a plurality of numerical values. That is, the feature amount 60 is multi-dimensional data. The number of dimensions of the feature amount 60 is, for example, 512, 1024, 2048, or the like. The first feature amount 601, the second feature amount 602, and a third feature amount 603 (see Fig. 11) which will be described below have the same number of dimensions and can be compared in the same feature amount space 80 (see Fig. 12 and the like).

For example, as illustrated in Fig. 9, in a learning phase before the encoder unit 71 is used as the feature amount extractor 41, a third patch image 653L for learning is input to the autoencoder 70 to train the autoencoder 70. The autoencoder 70 outputs a restored image 73L for learning for the third patch image 653L for learning. The loss calculation of the autoencoder 70 using a loss function is performed on the basis of the third patch image 653L for learning and the restored image 73L for learning. Then, the update of various coefficients (for example, coefficients of convolutional layer filters) of the autoencoder 70 is set according to the results of the loss calculation, and the autoencoder 70 is updated according to the update setting.

In the learning phase of the autoencoder 70, the series of processes of the input of the third patch image 653L for learning to the autoencoder 70, the output of the restored image 73L for learning from the autoencoder 70, the loss calculation, the update setting, and the update of the autoencoder 70 is repeatedly performed while the third patch image 653L for learning is exchanged. The repetition of the series of processes is ended in a case where the accuracy of the restoration from the third patch image 653L for learning to the restored image 73L for learning reaches a predetermined setting level. The encoder unit 71 of the autoencoder 70 whose restoration accuracy has reached the setting level in this way is stored as the feature amount extractor 41 in the storage 30 of the drug discovery support apparatus 10. In addition, in a case where the series of processes is repeated a set number of times, the training may be ended, regardless of the accuracy of the restoration from the third patch image 653L for learning to the restored image 73L for learning.

This training of the autoencoder 70 may be performed by the drug discovery support apparatus 10 or may be performed by an apparatus different from the drug discovery support apparatus 10. In the latter case, the feature amount extractor 41 is transmitted from another apparatus to the drug discovery support apparatus 10, and the RW control unit 50 stores the feature amount extractor 41 in the storage 30.

For example, as illustrated in Fig. 10, the third patch image 653L for learning is supplied from a plurality of third patch images 653 obtained by subdividing the liver section LVS included in a third section image 153. The third section image 153 is an image obtained by imaging the liver section LVS of the subject S in a past control group 75. The past control group 75 is composed of a plurality of subjects S to which the candidate substance for a drug was not administered in the past evaluation test. Therefore, the number of subjects S constituting the past control group 75 is significantly larger than the number of subjects S constituting the current administration group 25 and the current control group 27 and is, for example, about hundreds to thousands. Similarly to the first section image 151 and the like, a plurality of third section images 153 are obtained from one subject S. Therefore, the number of third section images 153 obtained from the past control group 75 is a value obtained by multiplying the number of third section images 153 obtained from one subject S by the number of subjects S.

Next, the structure of the third feature amount information 42 will be described. First, for example, as illustrated in Fig. 11, the feature amount extractor 41 extracts a plurality of third feature amounts 603 from each of a plurality of third patch images 653 based on a plurality of third section images 153 obtained from all of the subjects S constituting the past control group 75.

Fig. 12 illustrates an example of a graph in which the plurality of third feature amounts 603 extracted in Fig. 11 are plotted in the feature amount space 80. The third feature amount information 42 includes the coordinates (hereinafter, referred to as representative position coordinates) 81 of a representative position of the third feature amount 603 represented by an x mark in the feature amount space 80. The representative position is, for example, a center point or an average point of a distribution 82 of the third feature amounts 603. In addition, in Fig. 12, for convenience of explanation, the dimensions of the feature amount space 80 are two dimensions having a D1-axis and a D2-axis. However, the actual dimensions of the feature amount space 80 are, for example, 512 dimensions as described above. Similarly, in Fig. 13 and the like which will be described below, the dimensions of the feature amount space 80 are described as two dimensions for convenience of explanation.

Similarly to the training of the autoencoder 70, the third feature amount information 42 may be created by the drug discovery support apparatus 10 or may be created by an apparatus different from the drug discovery support apparatus 10. In the latter case, the third feature amount information 42 is transmitted from another apparatus to the drug discovery support apparatus 10, and the RW control unit 50 stores the third feature amount information 42 in the storage 30. In addition, the third feature amount information 42 may be the plurality of third feature amounts 603. In this case, the drug discovery support apparatus 10 derives the representative position coordinates 81.

For example, as illustrated in Fig. 13, a distribution of the first feature amounts 601 extracted for each of a plurality of first patch images 651 obtained by subdividing one target first section image 151T is surrounded by a two-dot chain line and is represented by reference numeral 85. In addition, a distribution of the second feature amounts 602 extracted for each of a plurality of second patch images 652 obtained by subdividing all of the second section images 152 is surrounded by a two-dot chain line and is represented by reference numeral 86.

The determination unit 52 calculates a first distance D1 between the representative position of the third feature amount 603 indicated by the representative position coordinates 81 of the third feature amount information 42 and the position of the first feature amount 601 in the feature amount space 80. The determination unit 52 calculates the first distances D1 of a plurality of first feature amounts 601 extracted for each of a plurality of first patch images 651 obtained by subdividing one target first section image 151T. In addition, the determination unit 52 calculates a second distance D2 between the representative position of the third feature amount 603 and the position of the second feature amount 602 in the feature amount space 80. The determination unit 52 calculates the second distances D2 of a plurality of second feature amounts 602 extracted for each of a plurality of second patch images 652 obtained by subdividing all of the second section images 152. The first distance D1 and the second distance D2 are Mahalanobis distances.

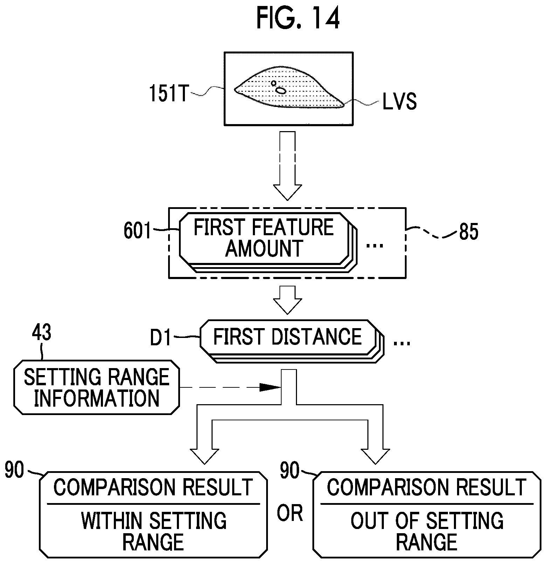

For example, as illustrated in Fig. 14, the determination unit 52 compares the calculated first distance D1 with the setting range of the setting range information 43. The setting range information 43 includes one threshold value indicating the setting range. In a case where the first distance D1 is less than the threshold value of the setting range information 43, the determination unit 52 outputs a comparison result 90 indicating that the first distance D1 is within the setting range. On the other hand, in a case where the first distance D1 is equal to or greater than the threshold value of the setting range information 43, the determination unit 52 outputs a comparison result 90 indicating that the first distance D1 is out of the setting range.

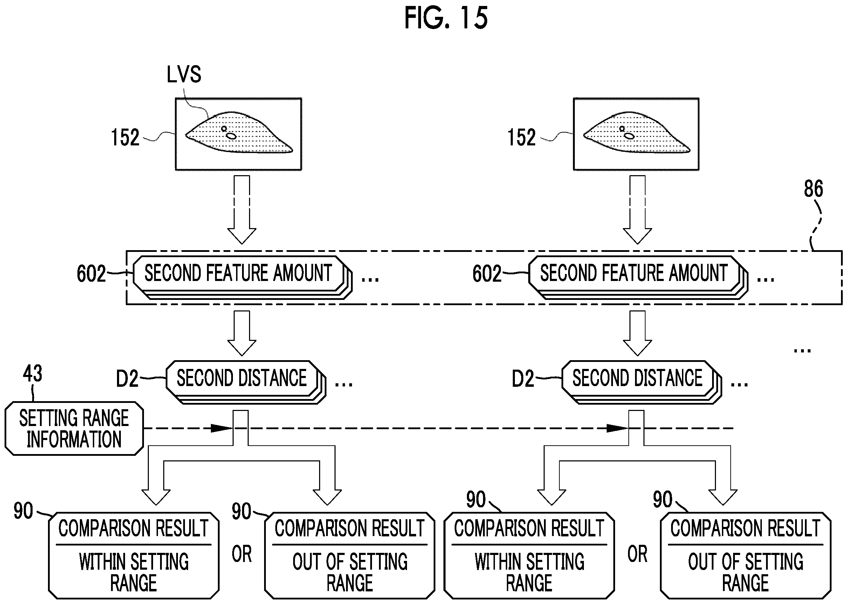

For example, as illustrated in Fig. 15, the determination unit 52 compares the calculated second distance D2 with the setting range of the setting range information 43. In a case where the second distance D2 is less than the threshold value of the setting range information 43, the determination unit 52 outputs a comparison result 90 indicating that the second distance D2 is within the setting range. On the other hand, in a case where the second distance D2 is equal to or greater than the threshold value of the setting range information 43, the determination unit 52 outputs a comparison result 90 indicating that the second distance D2 is out of the setting range.

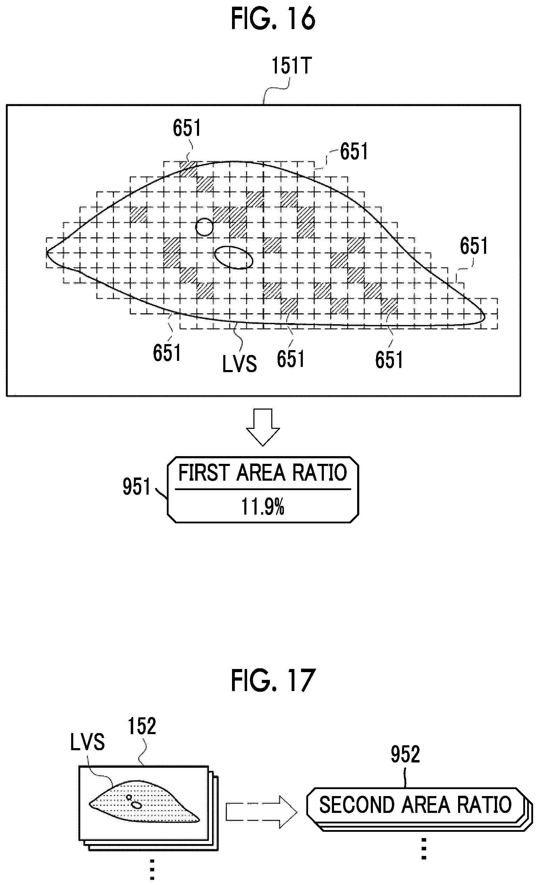

For example, as illustrated in Fig. 16, the determination unit 52 calculates a first area ratio 951 of the first patch images 651 (hatched), for which the first distance D1 is out of the setting range, to the target first section image 151T. Specifically, the determination unit 52 divides the number of first patch images 651, for which the first distance D1 is out of the setting range, by the total number of first patch images 651 to calculate the first area ratio 951. Fig. 16 illustrates a case where the first area ratio 951 is 11.9%.

As in the case of the first area ratio 951, the determination unit 52 calculates a second area ratio 952 of the second patch images 652, for which the second distance D2 is out of the setting range, to the second section image 152. However, since the second distance D2 is calculated on the basis of all of the second section images 152 as described above, the determination unit 52 calculates the second area ratio 952 for each of the second section images 152 as illustrated in Fig. 17 as an example.

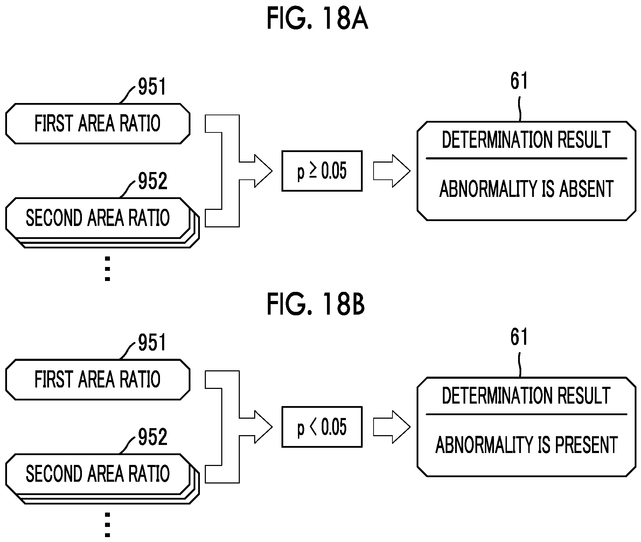

For example, as illustrated in Figs. 18A and 18B, the determination unit 52 determines whether or not the abnormality caused by the candidate substance 26 is present in the liver section LVS included in the target first section image 151T, on the basis of a p-value in statistical hypothesis testing based on a null hypothesis in which there is no difference between the first area ratio 951 and the second area ratio 952. As illustrated in Fig. 18A, in a case where the p-value is equal to or greater than a significance level (here, 0.05 is given as an example), the determination unit 52 determines that there is no statistically significant difference and outputs a determination result 61 indicating that the abnormality caused by the candidate substance 26 is not present in the liver section LVS included in the target first section image 151T. On the other hand, as illustrated in Fig. 18B, in a case where the p-value is less than the significance level, the determination unit 52 determines that there is a statistically significant difference and outputs a determination result 61 indicating that the abnormality caused by the candidate substance 26 is present in the liver section LVS included in the target first section image 151T.

For example, as illustrated in Fig. 19, the display control unit 53 performs control to display a drug discovery support screen 100 on the display 11. In a case where the operator inputs an instruction to display the first section image 151 through the input device 12 and the instruction receiving unit 110 receives the display instruction, the display control unit 53 displays the drug discovery support screen 100. A plurality of first section images 151 are displayed side by side on the drug discovery support screen 100. The plurality of first section images 151 are the first section images 151 obtained from one subject S among a plurality of subjects S constituting the current administration group 25. Fig. 19 illustrates an example in which 10 first section images 151 with image IDs "SP001" to "SP010" obtained from the subject S whose subject ID is "R001" are displayed side by side. In addition, the subject ID is displayed in the form of a pull-down menu 101, and it is possible to switch the subject S for which the first section images 151 are displayed on the drug discovery support screen 100. Further, the pull-down menu 101 has an option for designating all of the plurality of subjects S constituting the current administration group 25.

An abnormality determination button 102 is provided in a lower portion of the drug discovery support screen 100. In a case where the operator selects the abnormality determination button 102, the instruction receiving unit 110 receives an abnormality determination instruction. The abnormality determination instruction is an instruction for causing the first feature amount extraction unit 511, the second feature amount extraction unit 512, and the determination unit 52 to extract the first feature amount 601 and the second feature amount 602 and to determine whether or not an abnormality is present in the liver section LVS included in the first section image 151 displayed on the drug discovery support screen 100. That is, the first section image 151 displayed on the drug discovery support screen 100 corresponds to the target first section image 151T.

In a case where the abnormality determination button 102 has been selected and the determination unit 52 has determined whether or not an abnormality is present in the liver section LVS included in the target first section image 151T, for example, the display control unit 53 displays the determination result 61 below each first section image 151 as illustrated in Fig. 20. Fig. 20 illustrates a case where it has been determined that an abnormality is present in the liver section LVS included in three first section images 151 with image IDs "SP007" to "SP009". In addition, a frame indicating the first patch image 651, for which the comparison result 90 indicates that the distance is out of the setting range, may be displayed to be superimposed on the first section image 151.

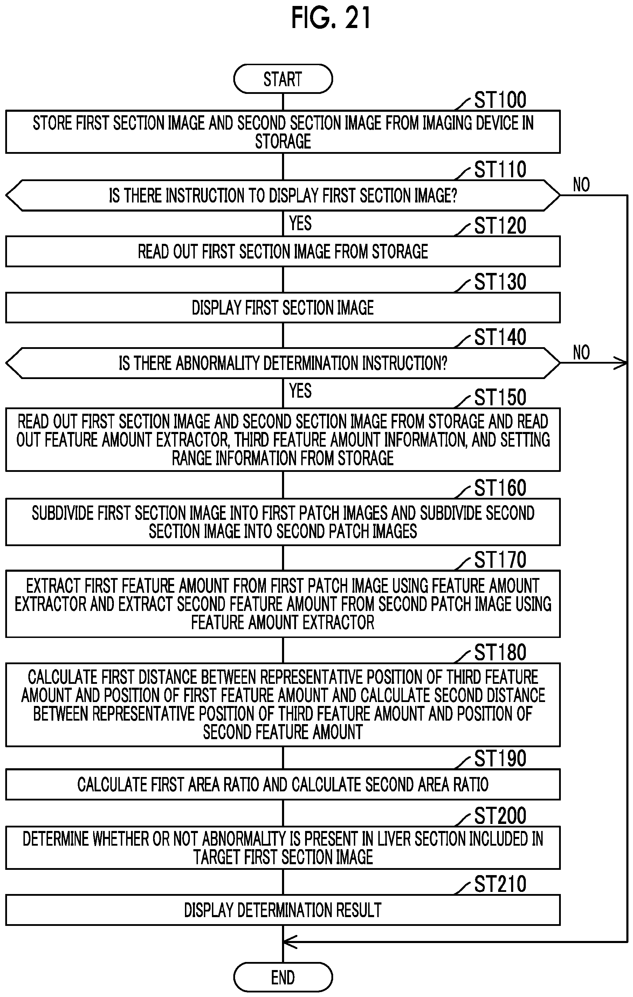

Next, the operation of the above-described configuration will be described with reference to, for example, a flowchart illustrated in Fig. 21. First, in a case where the operation program 40 is started in the drug discovery support apparatus 10, the CPU 32 of the drug discovery support apparatus 10 functions as the RW control unit 50, the first feature amount extraction unit 511, the second feature amount extraction unit 512, the determination unit 52, and the display control unit 53 as illustrated in Fig. 4.

First, the imaging device 19 captures the first section image 151 obtained by imaging the liver section LVS of the subject S in the current administration group 25 and the second section image 152 obtained by imaging the liver section LVS of the subject S in the current control group 27. The first section image 151 and the second section image 152 are output from the imaging device 19 to the drug discovery support apparatus 10. In the drug discovery support apparatus 10, the RW control unit 50 stores the first section image 151 and the second section image 152 from the imaging device 19 in the storage 30 (Step ST100).

In a case where the operator inputs an instruction to display the first section image 151 through the input device 12 (YES in Step ST110), the RW control unit 50 reads out the first section image 151 designated by the display instruction from the storage 30 (Step ST120). The first section image 151 is output from the RW control unit 50 to the display control unit 53. As illustrated in Fig. 19, the first section image 151 is displayed on the display 11 through the drug discovery support screen 100 under the control of the display control unit 53 (Step ST130).

In a case where the operator selects the abnormality determination button 102 on the drug discovery support screen 100 to issue an abnormality determination instruction (YES in Step ST140), the RW control unit 50 reads out the first section image 151 displayed on the drug discovery support screen 100 at that time as the target first section image 151T from the storage 30 to acquire the first section image 151 (Step ST150). In addition, the RW control unit 50 reads out all of the second section images 152 from the storage 30 to acquire the second section images 152 (Step ST150). The target first section image 151T is output from the RW control unit 50 to the first feature amount extraction unit 511. Further, the second section image 152 is output from the RW control unit 50 to the second feature amount extraction unit 512.

The feature amount extractor 41 is read out from the storage 30 by the RW control unit 50 (Step ST150) and is output to the first feature amount extraction unit 511 and the second feature amount extraction unit 512. In addition, the third feature amount information 42 is read out from the storage 30 by the RW control unit 50 to be acquired (Step ST150) and is output to the determination unit 52. Further, the setting range information 43 is read out from the storage 30 by the RW control unit 50 (Step ST150) and is output to the determination unit 52.

As illustrated in Fig. 5, the first feature amount extraction unit 511 subdivides the first section image 151 into a plurality of first patch images 651 (Step ST160). Similarly, the second feature amount extraction unit 512 subdivides the second section image 152 into a plurality of second patch images 652 (Step ST160).

Then, as illustrated in Fig. 6, the first feature amount extraction unit 511 extracts the first feature amount 601 from the first patch image 651 using the feature amount extractor 41 (Step ST170). The first feature amount 601 is output from the first feature amount extraction unit 511 to the determination unit 52. In addition, as illustrated in Fig. 7, the second feature amount extraction unit 512 extracts the second feature amount 602 from the second patch image 652 using the feature amount extractor 41 (Step ST170). The second feature amount 602 is output from the second feature amount extraction unit 512 to the determination unit 52.

As illustrated in Fig. 13, the determination unit 52 calculates the first distance D1 between the representative position of the third feature amount 603 and the position of the first feature amount 601 (Step ST180). In addition, the second distance D2 between the representative position of the third feature amount 603 and the position of the second feature amount 602 is calculated (Step ST180).

As illustrated in Fig. 14, the determination unit 52 compares the first distance D1 with the setting range of the setting range information 43 and outputs the comparison result 90. In addition, as illustrated in Fig. 15, the determination unit 52 compares the second distance D2 with the setting range of the setting range information 43 and outputs the comparison result 90. Then, as illustrated in Fig. 16, the first area ratio 951 of the first patch image 651, for which the first distance D1 is out of the setting range, to the first section image 151 is calculated (Step ST190). Further, as illustrated in Fig. 17, the second area ratio 952 of the second patch image 652, for which the second distance D2 is out of the setting range, to the second section image 152 is calculated (Step ST190).

As illustrated in Fig. 18, the determination unit 52 determines whether or not the abnormality caused by the candidate substance 26 is present in the liver section LVS included in the target first section image 151T, on the basis of the p-value in the statistical hypothesis testing using the first area ratio 951 and the second area ratio 952 (Step ST200). The determination result 61 is output from the determination unit 52 to the display control unit 53. As illustrated in Fig. 20, the determination result 61 is displayed on the display 11 through the drug discovery support screen 100 under the control of the display control unit 53 (Step ST210). The operator views the determination result 61 and performs the evaluation of the candidate substance 26 based on the determination result 61 such as the detailed observation of the first section image 151 determined to be abnormal.

As described above, the CPU 32 of the drug discovery support apparatus 10 comprises the RW control unit 50, the first feature amount extraction unit 511, the second feature amount extraction unit 512, and the determination unit 52. The RW control unit 50 reads out the first section image 151, the second section image 152, and the third feature amount information 42 from the storage 30 to acquire them. The first section image 151 is an image obtained by imaging the liver section LVS of the subject S constituting the current administration group 25 to which the candidate substance 26 for a drug has been administered. The second section images 152 are a plurality of images obtained by imaging the liver sections LVS of a plurality of subjects S that have the same attributes and are placed in the same breeding environment as the subjects S constituting the current administration group 25 and that constitute the current control group 27 to which the candidate substance 26 for a drug has not been administered. The third feature amount information 42 is information related to the third feature amount 603 extracted from the third section image 153. The third section images 153 are a plurality of images obtained by imaging the liver sections LVS of a plurality of subjects S constituting the past control group 75 to which the candidate substance was not administered in the past evaluation test.

The first feature amount extraction unit 511 extracts the first feature amount 601 from the target first section image 151T. The second feature amount extraction unit 512 extracts the second feature amount 602 from each of the plurality of second section images 152. The determination unit 52 determines whether or not an abnormality is present in the liver section LVS included in the target first section image 151T on the basis of the first feature amount 601, the second feature amount 602, and the third feature amount information 42.

The current control group 27 includes the subject S in which an abnormality has occurred due to the attribute and/or the breeding environment. The same applies to the past control group 75. In the technology of the present disclosure, the second feature amount 602 and the third feature amount information 42 based on the current control group 27 and the past control group 75 are compared with the first feature amount 601 based on the current administration group 25 to determine whether or not an abnormality is present in the liver section LVS of the subject S constituting the current administration group 25. Therefore, in a case where the determination result 61 output by this determination indicates that an abnormality is present, it can be seen that the cause of the abnormality is not the attribute and/or the breeding environment, in other words, that the cause of the abnormality is the candidate substance 26. Therefore, according to the technology of the present disclosure, it is possible to determine whether or not the abnormality caused by the candidate substance 26 for a drug has occurred in the subject S in the current administration group 25.

The subjects S constituting the current control group 27 have the same attributes and are placed in the same breeding environment as the subjects S constituting the current administration group 25. However, since the number of subjects S constituting the current control group 27 is small, it is difficult to perform the determination on the basis of only the number of subjects S constituting the current control group 27. Therefore, in the technology of the present disclosure, the small number of subjects S constituting the current control group 27 is compensated for by the past control group 75. On the other hand, the number of subjects S constituting the past control group 75 is large, but the subjects S constituting the past control group 75 may have attributes and breeding environments different from those of the subjects S constituting the current administration group 25. As a result, in a case where the determination is performed on the basis of only the number of subjects S constituting the past control group 75, there is a concern that there will be many erroneous determinations. Therefore, in the technology of the present disclosure, the differences in the attributes and breeding environments of the subjects S constituting the past control group 75 are compensated for by the current control group 27. That is, the current control group 27 and the past control group 75 have a complementary relationship. In the technology of the present disclosure, the current control group 27 and the past control group 75 having the complementary relationship therebetween can be appropriately combined to determine whether or not the cause of the abnormality occurring in the subject S is the candidate substance 26.

The first feature amount extraction unit 511 and the second feature amount extraction unit 512 extract the first feature amount 601 and the second feature amount 602, using the feature amount extractor 41 used as the encoder unit 71 of the autoencoder 70 trained using a plurality of third section images 153. Therefore, it is possible to easily extract the first feature amount 601 and the second feature amount 602 that well represent the shape and texture features of the liver section LVS.

The first feature amount 601, the second feature amount 602, and the third feature amount 603 have the same number of dimensions and can be compared in the same feature amount space 80. The third feature amount information 42 is information of the representative positions of a plurality of third feature amounts 603 in the feature amount space 80. The determination unit 52 calculates the first distance D1 between the representative position of the third feature amount 603 and the position of the first feature amount 601 in the feature amount space 80. In addition, the determination unit 52 calculates the second distance D2 between the representative position of the third feature amount 603 and the position of each of a plurality of second feature amounts 602 in the feature amount space 80. The determination unit 52 performs the determination on the basis of the first distance D1 and the second distance D2.

The first distance D1 indicates the degree of deviation between the target first section image 151T and the third section image 153, and the second distance D2 indicates the degree of deviation between the second section image 152 and the third section image 153. The degree of deviation between the target first section image 151T and the third section image 153 indicates the degree of difference between the abnormality occurring in the current administration group 25 and the abnormality occurring in the past control group 75. In addition, the degree of deviation between the second section image 152 and the third section image 153 indicates the degree of difference between the abnormality occurring in the current control group 27 and the abnormality occurring in the past control group 75. Therefore, in a case where the determination is performed on the basis of the first distance D1 and the second distance D2, it is possible to perform the determination, considering both the degree of difference between the abnormality occurring in the current administration group 25 and the abnormality occurring in the past control group 75 and the degree of difference between the abnormality occurring in the current control group 27 and the abnormality occurring in the past control group 75. As a result, it is possible to increase the accuracy of the determination.

The first feature amount extraction unit 511 extracts the first feature amount 601 for each of a plurality of first patch images 651 obtained by subdividing the target first section image 151T. In addition, the second feature amount extraction unit 512 extracts the second feature amount 602 for each of a plurality of second patch images 652 obtained by subdividing the second section image 152. The determination unit 52 calculates the first distance D1 for each of a plurality of first feature amounts 601 extracted from the plurality of first patch images 651. Further, the determination unit 52 calculates the second distance D2 for each of a plurality of second feature amounts 602 extracted from the plurality of second patch images 652. The determination unit 52 performs the determination on the basis of the first area ratio 951 of the first patch images 651, for which the first distance D1 is out of the setting range, to the target first section image 151T and the second area ratio 952 of the second patch images 652, for which the second distance D2 is out of the setting range, to the second section image 152. The amount of information used as a reference for determination increases significantly, as compared to a case where the determination is performed without subdividing the target first section image 151T and the second section image 152 into the first patch images 651 and the second patch images 652. Therefore, it is possible to further increase the accuracy of the determination.

The value of the first area ratio 951 is larger as the degree of difference between the abnormality occurring in the current administration group 25 and the abnormality occurring in the past control group 75 is larger (the abnormality occurring in the current administration group 25 is more caused by the candidate substance 26). On the other hand, the value of the second area ratio 952 is smaller as the degree of difference between the abnormality occurring in the current control group 27 and the abnormality occurring in the past control group 75 is smaller (the abnormality occurring in the current control group 27 is more similar to the abnormality occurring in the past control group 75). Therefore, in a case where it is considered that there is a statistically significant difference between the first area ratio 951 and the second area ratio 952, it can be said that the abnormality caused by the candidate substance 26 is highly likely to be present in the liver section LVS included in the target first section image 151T. Therefore, in a case where there is a statistically significant difference between the first area ratio 951 and the second area ratio 952, it is determined that an abnormality is present in the liver section LVS included in the target first section image 151T, which makes it possible to further increase the accuracy of the determination.

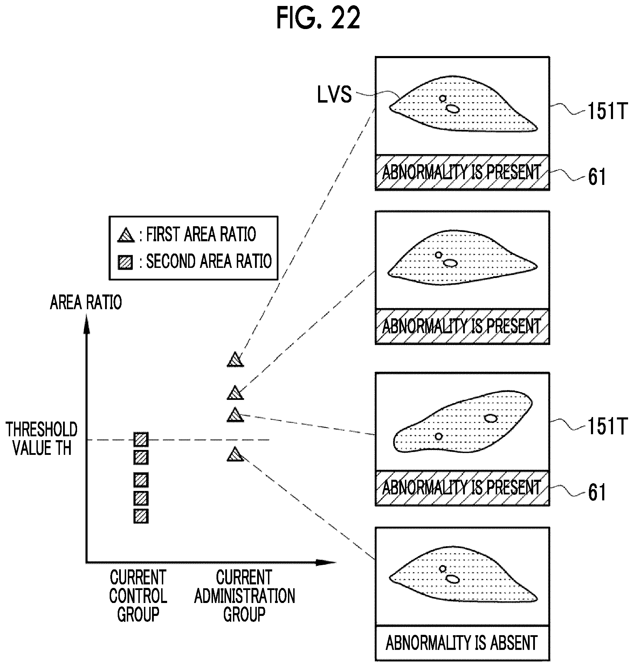

In addition, a criterion for determination may be, for example, a threshold value TH set on the basis of the second area ratio 952 as illustrated in Fig. 22 as an example. In Fig. 22, the maximum value of a plurality of second area ratios 952 calculated on the basis of the plurality of second section images 152 is given as an example of the threshold value TH. In this case, the determination unit 52 determines that the abnormality caused by the candidate substance 26 is present in the liver section LVS included in the target first section image 151T for which the first area ratio 951 is greater than the threshold value TH. According to this aspect, since there is no need to derive the p-value or to compare the p-value with the significance level, it is possible to easily complete the determination. In addition, the threshold value TH may be, for example, an average value or a mode value of the plurality of second area ratios 952.

### [2_1-th Embodiment]

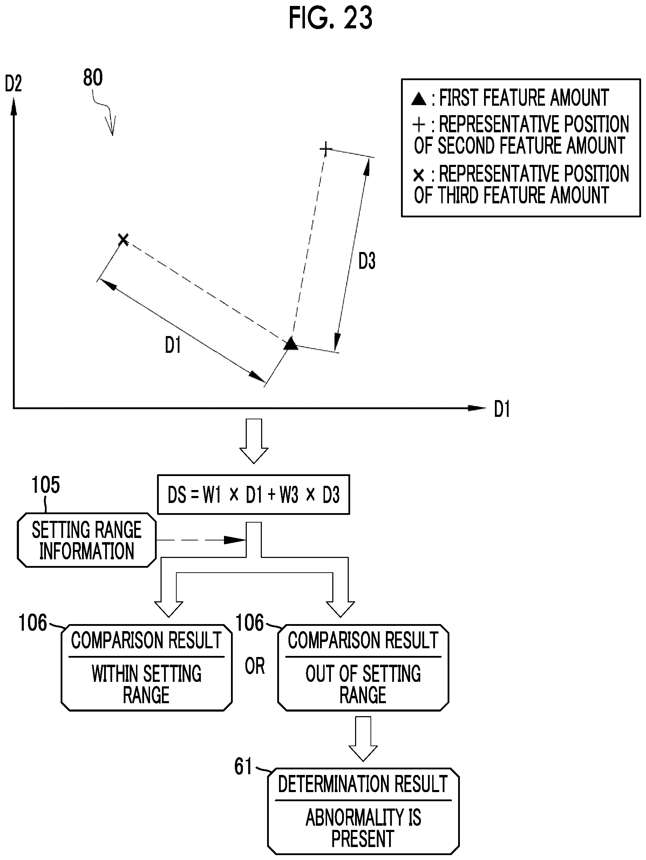

For example, as illustrated in Fig. 23, in a 2_1-th embodiment, the determination unit 52 calculates a third distance D3 instead of the second distance D2. The third distance D3 is a distance between the representative position (indicated by a + mark) of a plurality of second feature amounts 602 and the position of the first feature amount 601 in the feature amount space 80. The third distance D3 is also the Mahalanobis distance. The representative position of the second feature amounts 602 is a center point, an average point, or the like of a distribution 86 of the second feature amounts 602 illustrated in Fig. 13.

The determination unit 52 calculates a weighted sum DS = W1 × D1 + W3 × D3 of the first distance D1 and the third distance D3. Here, W1 is a weighting coefficient by which the first distance D1 is multiplied, and W3 is a weighting coefficient by which the third distance D3 is multiplied. 0 ≤ W1 ≤ 1 and 0 ≤ W3 ≤ 1 are established, and W1 + W3 = 1 is established. The determination unit 52 compares the weighted sum DS with a setting range of setting range information 105. The setting range information 105 is stored in the storage 30 and includes one threshold value indicating the setting range. In a case where the weighted sum DS is less than the threshold value of the setting range, the determination unit 52 outputs a comparison result 106 indicating that the weighted sum DS is within the setting range. On the other hand, in a case where the weighted sum DS is equal to or greater than the threshold value of the setting range, the determination unit 52 outputs a comparison result 106 indicating that the weighted sum DS is out of the setting range.

The determination unit 52 calculates the first distance D1, the third distance D3, and the weighted sum DS for each of the first feature amounts 601 constituting the distribution 85 illustrated in Fig. 13 and outputs the comparison result 106. In a case where the comparison result 106 indicates that any one of the weighted sums DS calculated from the plurality of first feature amounts 601 constituting the distribution 85 is out of the setting range, the determination unit 52 outputs a determination result 61 indicating that the abnormality caused by the candidate substance 26 is present in the liver section LVS included in the target first section image 151T.

As described above, in the 2_1-th embodiment, the determination unit 52 calculates the first distance D1 between the representative position of the third feature amount 603 and the position of the first feature amount 601 in the feature amount space 80 and calculates the third distance D3 between the representative position of a plurality of second feature amounts 602 and the position of the first feature amount 601 in the feature amount space 80. Then, the determination unit 52 performs the determination on the basis of the first distance D1 and the third distance D3.

As described above, the first distance D1 indicates the degree of deviation between the target first section image 151T and the third section image 153, and the degree of deviation between the target first section image 151T and the third section image 153 indicates the degree of difference between the abnormality occurring in the current administration group 25 and the abnormality occurring in the past control group 75. In addition, the third distance D3 indicates the degree of deviation between the target first section image 151T and the second section image 152, and the degree of deviation between the target first section image 151T and the second section image 152 indicates the degree of difference between the abnormality occurring in the current administration group 25 and the abnormality occurring in the current control group 27. Therefore, in a case where the determination is performed on the basis of the first distance D1 and the third distance D3, it is possible to perform the determination, considering both the degree of difference between the abnormality occurring in the current administration group 25 and the abnormality occurring in the past control group 75 and the degree of difference between the abnormality occurring in the current administration group 25 and the abnormality occurring in the current control group 27. As a result, it is possible to increase the accuracy of the determination. However, in the 2_1-th embodiment, it is necessary to derive the representative position of the second feature amounts 602. Therefore, in the first embodiment that does not require the derivation, it takes a small amount of time and effort to perform the process.

In a case where the weighted sum DS of the first distance D1 and the third distance D3 is out of the setting range, the determination unit 52 determines that an abnormality is present in the liver section LVS included in the target first section image 151T. Therefore, it is possible to perform the determination, considering both the degree of difference between the abnormality occurring in the current administration group 25 and the abnormality occurring in the past control group 75 and the degree of difference between the abnormality occurring in the current administration group 25 and the abnormality occurring in the current control group 27. As a result, it is possible to increase the accuracy of the determination.

In addition, the determination may be performed on the basis of a simple sum D1 + D3 of the first distance D1 and the third distance D3 instead of the weighted sum DS. Further, in a case where the area ratio of the first patch images 651, for which the comparison result 106 is out of the setting range, to the first section image 151 is equal to or greater than the threshold value, it may be determined that an abnormality is present in the liver section LVS included in the first section image 151.

### [2_2-th Embodiment]

For example, as illustrated in Fig. 24, in a 2_2-th embodiment, the instruction receiving unit 110 receives a coefficient setting instruction 111 from the operator through the input device 12. The coefficient setting instruction 111 is an instruction for setting weighting coefficients W1 and W3 and includes the weighting coefficients W1 and W3 set by the operator. In a case where the instruction receiving unit 110 receives the coefficient setting instruction 111, the instruction receiving unit 110 outputs coefficient setting information 112 to the determination unit 52. The coefficient setting information 112 is information obtained by directly copying the weighting coefficients W1 and W3 included in the coefficient setting instruction 111. The determination unit 52 calculates a weighted sum DS, using the weighting coefficients W1 and W3 of the coefficient setting information 112. Fig. 24 illustrates an example in which the weighting coefficient W1 is set to 0.2 and the weighting coefficient W3 is set to 0.8.

In a case where the current evaluation test related to the current administration group 25 and the current control group 27 and the past evaluation test related to the past control group 75 have similar content, the operator sets the weighting coefficients such that the weighting coefficient W1 is larger than the weighting coefficient W3. On the other hand, in a case where the current evaluation test related to the current administration group 25 and the current control group 27 and the past evaluation test related to the past control group 75 have different content, the operator sets the weighting coefficients such that the weighting coefficient W3 is larger value than the weighting coefficient W1.

As described above, in the 2_2-th embodiment, the instruction receiving unit 110 receives the coefficient setting instruction 111 which is an instruction to set the weighting coefficients W1 and W3 that are multiplied by the first distance D1 and the third distance D3 in the weighted sum DS. Therefore, the operator can set the weighting coefficients W1 and W3 adapted to the similarity between the current evaluation test related to the current administration group 25 and the current control group 27 and the past evaluation test related to the past control group 75. As a result, it is possible to increase the reliability of the determination result 61.

In addition, the similarity between the current evaluation test related to the current administration group 25 and the current control group 27 and the past evaluation test related to the past control group 75 may be derived by the comparison between the test conditions of the current evaluation test and the test conditions of the past evaluation test, and the weighting coefficients W1 and W3 adapted to the derived similarity may be automatically set.

### [Third Embodiment]

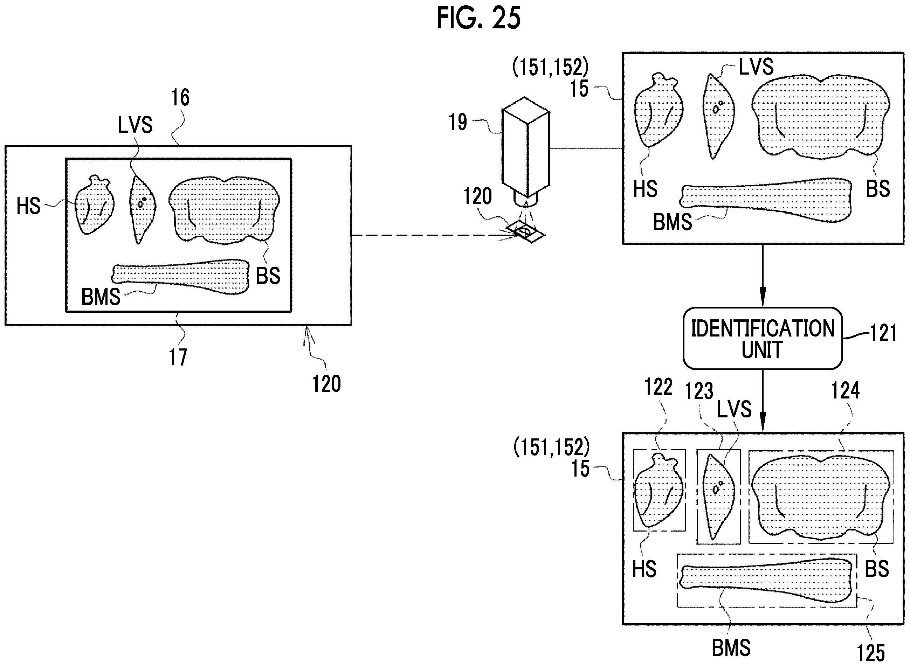

For example, as illustrated in Fig. 25, in a third embodiment, a slide specimen 120 in which tissue sections of a plurality of types of organs are placed on one slide glass 16 is handled. Fig. 25 illustrates a case where a heart section HS, a brain section BS, and a bone marrow section BMS are placed in addition to the liver section LVS. In this case, the section image 15 (the first section image 151 and the second section image 152) includes the heart section HS, the brain section BS, and the bone marrow section BMS in addition to the liver section LVS.

The CPU 32 of the drug discovery support apparatus 10 according to the third embodiment functions as an identification unit 121 in addition to each of the processing units 50 to 53 according to the first embodiment. For example, the identification unit 121 identifies tissue sections of each organ from the section image 15 (the first section image 151 and the second section image 152), using a machine learning model and a template for identifying tissue sections of each organ. The identification unit 121 outputs coordinate information of frames 122 to 125 surrounding the tissue sections of each organ as identification results. The frame 122 is a frame surrounding the heart section HS, and the frame 123 is a frame surrounding the liver section LVS. In addition, the frame 124 is a frame surrounding the brain section BS, and the frame 125 is a frame surrounding the bone marrow section BMS.

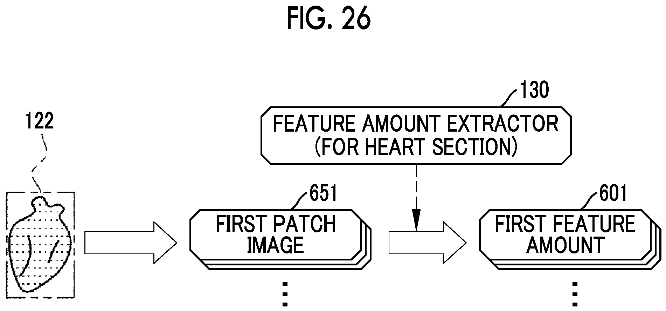

Fig. 26 illustrates an aspect in which the first feature amount extraction unit 511 subdivides the heart section HS in the frame 122 into a plurality of first patch images 651 and the first feature amount 601 is extracted from the first patch image 651 by a feature amount extractor 130 for the heart section HS. The first feature amount extraction unit 511 also extracts the first feature amounts 601 for the liver section LVS in the frame 123, the brain section BS in the frame 124, and the bone marrow section BMS in the frame 125. The second feature amount extraction unit 512 similarly extracts the second feature amount 602. That is, the first feature amount extraction unit 511 and the second feature amount extraction unit 512 extract the first feature amount 601 and the second feature amount 602 of each of the tissue sections of each organ identified by the identification unit 121. Since the subsequent process of the determination unit 52 is the same as the process described in the first embodiment or the 2_1-th embodiment except that the determination is performed on each of the tissue sections of each organ included in the target first section image 151T, the illustration and description thereof will not be repeated.

As described above, in the third embodiment, the first section image 151 and the second section image 152 are images obtained by imaging the slide specimen 120 on which the tissue sections of a plurality of types of organs are placed. The identification unit 121 identifies the tissue sections of each organ from the first section image 151 and the second section image 152. The first feature amount extraction unit 511 and the second feature amount extraction unit 512 extract the first feature amount 601 and the second feature amount 602 of each of the identified tissue sections of each organ. The determination unit 52 determines whether or not an abnormality is present in each of the tissue sections of each organ included in the target first section image 151T on the basis of the first feature amount 601, the second feature amount 602, and the third feature amount information 42. Therefore, it is also possible to respond to the slide specimen 120 on which the tissue sections of a plurality of types of organs are placed. For the slide specimen, the slide specimen 120 on which the tissue sections of a plurality of types of organs are placed as in the third embodiment is more general than the slide specimen 18 on which the tissue section of one organ is placed as in the first embodiment. Therefore, it is possible to perform drug discovery support that is more suitable for general operations.

A configuration may be adopted in which the operator manually defines the frames indicating the tissue sections of each organ in the first section image 151 and the second section image 152.

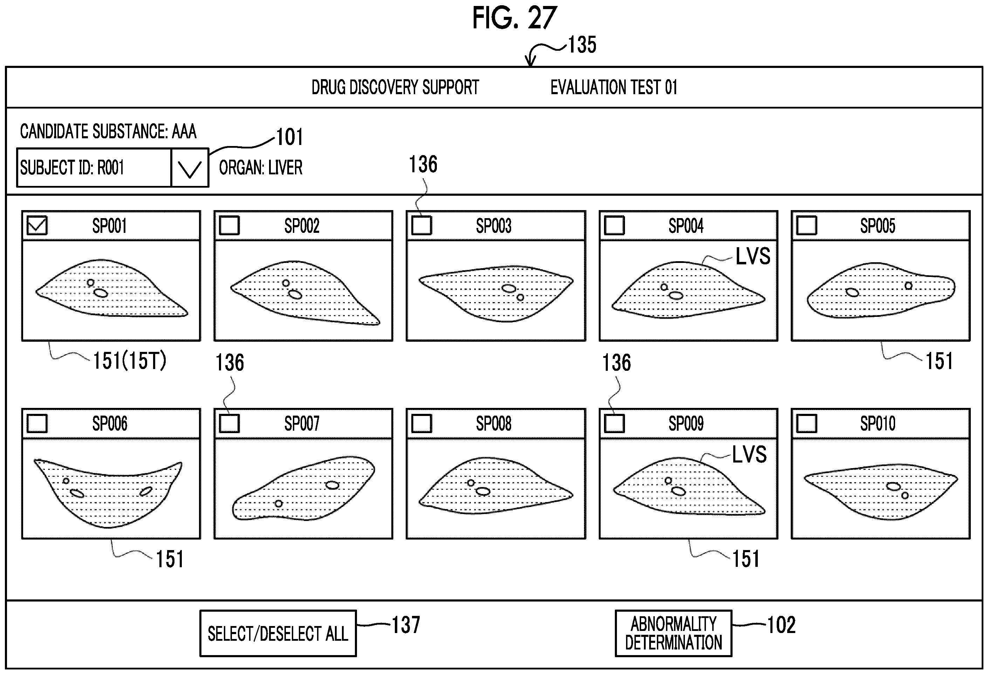

In addition, as in a drug discovery support screen 135 illustrated in Fig. 27 as an example, a check box 136 may be provided in each of a plurality of first section images 151 to be displayed such that the operator can select the first section image 151 to be used as the target first section image 151T. In addition to the abnormality determination button 102, a select/deselect all button 137 is provided in a lower portion of the drug discovery support screen 135. The select/deselect all button 137 is a button for selecting all of the plurality of first section images 151 as the target first section images 151T and is a button for canceling the selection. This enables the operator to select the target first section image 151T on which the operator wants to perform the determination. Fig. 27 illustrates a case where only the first section image 151 with the image ID "SP001" is selected. That is, the number of target first section images 151T to be subjected to the determination may be only one.

The encoder unit 71 of the autoencoder 70 is given as an example of the feature amount extractor 41. However, the present disclosure is not limited thereto. An encoder unit of a convolutional neural network which has been trained to output a determination result of whether or not an abnormality is present in a certain class, for example, the liver section LVS included in the patch image 65 in response to the input of the patch image 65 may be used as the feature amount extractor 41. In addition, another machine learning model, such as a support vector machine, may be used as the feature amount extractor 41.

In the tissue section included in the third patch image 653L for learning, a region that is likely to be erroneously detected as an abnormality may be masked and then used for learning. Examples of the region that is likely to be erroneously detected as an abnormality include a blood vessel region in the liver section LVS and dust stuck in a case where the slide specimen 18 or 120 is created.

The feature amount 60 is not limited to the feature amount extracted by the feature amount extractor 41. The feature amount 60 may be, for example, the average value, maximum value, minimum value, mode value, or variance of the pixel values of the patch image 65.

The organ is not limited to the liver LV or the like given as an example. The organ may be a stomach, a lung, a small intestine, a large intestine, or the like. In addition, the subject S is not limited to the rat. The subject S may be a mouse, a guinea pig, a sand mouse, a hamster, a ferret, a rabbit, a dog, a cat, a monkey, or the like.

The technique described in JP2021-508373A may be applied. It is determined that an abnormality has occurred in the tissue section of the subject S in the current administration group 25 by the technique described in JP2021-508373A. However, the technology of the present disclosure shows that, in a case where the content of the determination result 61 of the subject S is that there is no abnormality, the cause of the abnormality is not the candidate substance 26 but the attribute and the breeding environment.

The drug discovery support apparatus 10 may be a personal computer that is installed in a pharmaceutical facility as illustrated in Fig. 1 or may be a server computer that is installed in a data center independent of the pharmaceutical facility.

In a case where the drug discovery support apparatus 10 is configured by the server computer, the first section image 151 and the second section image 152 are transmitted from the personal computer installed in each pharmaceutical facility to the server computer via a network such as the Internet. The server computer delivers the drug discovery support screen 100 or 135 to the personal computer in the form of screen data for web delivery created by a markup language such as Extensible Markup Language (XML). The personal computer reproduces the drug discovery support screen 100 or 135 to be displayed on the web browser on the basis of the screen data and displays the drug discovery support screen 100 or 135 on the display. Further, instead of XML, another data description language, such as JavaScript (registered trademark) Object Notation (JSON), may be used.

The drug discovery support apparatus 10 according to the technology of the present disclosure can be widely used throughout all stages of drug development from the setting of a drug discovery target, which is the initial stage, to a clinical trial which is the final stage.

The hardware configuration of the computer constituting the drug discovery support apparatus 10 according to the technology of the present disclosure can be modified in various ways. For example, the drug discovery support apparatus 10 may be configured by a plurality of computers that are separated as hardware for the purpose of improving processing capability and reliability. For example, the functions of the first feature amount extraction unit 511 and the second feature amount extraction unit 512 and the functions of the determination unit 52 may be distributed to two computers. In this case, the drug discovery support apparatus 10 is configured by the two computers.

As described above, the hardware configuration of the computer of the drug discovery support apparatus 10 can be changed as appropriate depending on required performance such as processing capacity, safety, and reliability. Further, not only the hardware but also an application program, such as the operation program 40, can be duplicated or distributed and stored in a plurality of storages for the purpose of ensuring safety and reliability.

In each of the above-described embodiments, for example, the following various processors can be used as a hardware structure of processing units executing various processes, such as the RW control unit 50, the first feature amount extraction unit 511, the second feature amount extraction unit 512, the determination unit 52, the display control unit 53, the instruction receiving unit 110, and the identification unit 121. The various processors include, for example, the CPU 32 which is a general-purpose processor executing software (operation program 40) to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to execute a specific process.

One processing unit may be configured by one of the various processors or by a combination of two or more processors of the same type or different types (for example, a combination of a plurality of FPGAs and/or a combination of a CPU and an FPGA). Further, a plurality of processing units may be configured by one processor.

A first example of the configuration in which a plurality of processing units are configured by one processor is an aspect in which one processor is configured by a combination of one or more CPUs and software and functions as a plurality of processing units. A representative example of this aspect is a client computer or a server computer. A second example of the configuration is an aspect in which a processor that implements the functions of the entire system including a plurality of processing units using one integrated circuit (IC) chip is used. A representative example of this aspect is a system on chip (SoC). As described above, the various processing units are configured by using one or more of the above various processors as the hardware structure.

In addition, more specifically, an electric circuit (circuitry) in which circuit elements, such as semiconductor elements, are combined can be used as the hardware structure of these various processors.

In the technology of the present disclosure, the above-described various embodiments and/or various modification examples may be combined with each other as appropriate. In addition, it goes without saying that the present disclosure is not limited to each of the above-described embodiments, and various configurations can be adopted without departing from the gist of the present disclosure. Furthermore, the technology of the present disclosure extends to a storage medium that non-temporarily stores a program, in addition to the program.

The above descriptions and illustrations are detailed descriptions of portions related to the technology of the present disclosure and are merely examples of the technology of the present disclosure. For example, the above description of the configurations, functions, operations, and effects is the description of examples of the configurations, functions, operations, and effects of portions related to the technology of the present disclosure. Therefore, unnecessary portions may be deleted or new elements may be added or replaced in the above descriptions and illustrations without departing from the gist of the technology of the present disclosure. In addition, in the above descriptions and illustrations, the description of, for example, common technical knowledge that does not need to be particularly described to enable the implementation of the technology of the present disclosure is omitted in order to avoid confusion and facilitate the understanding of portions related to the technology of the present disclosure.

In the present specification, "A and/or B" is synonymous with "at least one of A or B". That is, "A and/or B" means only A, only B, or a combination of A and B. Further, in the present specification, the same concept as "A and/or B" is applied to a case where the connection of three or more matters is expressed by "and/or".

All of the publications, the patent applications, and the technical standards described in the present specification are incorporated by reference herein to the same extent as each individual document, each patent application, and each technical standard are specifically and individually stated to be incorporated by reference.

## Claims

1. A drug discovery support apparatus comprising:
a processor configured to:
acquire a first section image obtained by imaging a tissue section of at least one of a plurality of subjects constituting an administration group to which a candidate substance for a drug has been administered;
extract a first feature amount from the first section image;
acquire a plurality of second section images obtained by imaging tissue sections of a plurality of subjects that have the same attributes and are placed under the same breeding environment as the subjects constituting the administration group and that constitute a control group to which the candidate substance for a drug has not been administered;
extract a second feature amount from each of the plurality of second section images;
acquire third feature amount information related to a third feature amount extracted from each of a plurality of third section images obtained by imaging tissue sections of a plurality of subjects constituting a control group to which the candidate substance was not administered in a past evaluation test; and
determine whether or not an abnormality is present in the tissue section included in the first section image on the basis of the first feature amount, the second feature amount, and the third feature amount information.

2. The drug discovery support apparatus according to claim 1, wherein the processor is configured to:
extract the first feature amount and the second feature amount using a machine learning model trained using the plurality of third section images.

3. The drug discovery support apparatus according to claim 1 or 2,
wherein the first feature amount, the second feature amount, and the third feature amount have the same number of dimensions and are comparable in the same feature amount space,
the third feature amount information is information of a representative position of the plurality of third feature amounts in the feature amount space, and
the processor is configured to:
calculate a first distance between the representative position and a position of the first feature amount in the feature amount space;
calculate a second distance between the representative position and a position of each of the plurality of second feature amounts in the feature amount space; and
perform the determination on the basis of the first distance and the second distance.

4. The drug discovery support apparatus according to claim 3, wherein the processor is configured to:
extract the first feature amount for each of a plurality of first patch images obtained by subdividing the first section image and calculate the first distance for each of the plurality of first patch images;
extract the second feature amount for each of a plurality of second patch images obtained by subdividing the second section image and calculate the second distance for each of the plurality of second patch images; and
perform the determination on the basis of a first area ratio of the first patch images, for which the first distance is out of a setting range, to the first section image and a second area ratio of the second patch images, for which the second distance is out of the setting range, to the second section image.

5. The drug discovery support apparatus according to claim 4, wherein the processor is configured to:
determine that the abnormality is present in the tissue section included in the first section image in a case where there is a statistically significant difference between the first area ratio and the second area ratio.

6. The drug discovery support apparatus according to claim 4, wherein the processor is configured to:
determine that the abnormality is present in the tissue section included in the first section image for which the first area ratio is greater than a threshold value set on the basis of the second area ratio.

7. The drug discovery support apparatus according to claim 1 or 2,
wherein the first feature amount, the second feature amount, and the third feature amount have the same number of dimensions and are comparable in the same feature amount space,
the third feature amount information is information of a representative position of the plurality of third feature amounts in the feature amount space, and
the processor is configured to:
calculate a first distance between the representative position and a position of the first feature amount in the feature amount space;
calculate a third distance between a representative position of a plurality of the second feature amounts and the position of the first feature amount in the feature amount space; and
perform the determination on the basis of the first distance and the third distance.

8. The drug discovery support apparatus according to claim 7, wherein the processor is configured to:
determine that the abnormality is present in the tissue section included in the first section image in a case where a weighted sum of the first distance and the third distance is out of a setting range.

9. The drug discovery support apparatus according to claim 8, wherein the processor is configured to:
receive an instruction to set weighting coefficients, by which the first distance and the third distance are multiplied, in the weighted sum.

10. The drug discovery support apparatus according to any one of claims 1 to 9,
wherein the first section image and the second section image are images obtained by imaging a slide specimen on which tissue sections of a plurality of types of organs are placed, and
the processor is configured to:
identify the tissue sections of each organ from the first section image and the second section image;
extract the first feature amount and the second feature amount of each of the identified tissue sections of each organ; and
determine whether or not an abnormality is present in each of the tissue sections of each organ included in the first section image on the basis of the first feature amount, the second feature amount, and the third feature amount information.

11. A method for operating a drug discovery support apparatus, the method comprising:
acquiring a first section image obtained by imaging a tissue section of at least one of a plurality of subjects constituting an administration group to which a candidate substance for a drug has been administered;
extracting a first feature amount from the first section image;
acquiring a plurality of second section images obtained by imaging tissue sections of a plurality of subjects that have the same attributes and are placed under the same breeding environment as the subjects constituting the administration group and that constitute a control group to which the candidate substance for a drug has not been administered;
extracting a second feature amount from each of the plurality of second section images;
acquiring third feature amount information related to a third feature amount extracted from each of a plurality of third section images obtained by imaging tissue sections of a plurality of subjects constituting a control group to which the candidate substance was not administered in a past evaluation test; and
determining whether or not an abnormality is present in the tissue section included in the first section image on the basis of the first feature amount, the second feature amount, and the third feature amount information.

12. A program for operating a drug discovery support apparatus, the program causing a computer to execute a process comprising:
acquiring a first section image obtained by imaging a tissue section of at least one of a plurality of subjects constituting an administration group to which a candidate substance for a drug has been administered;
extracting a first feature amount from the first section image;
acquiring a plurality of second section images obtained by imaging tissue sections of a plurality of subjects that have the same attributes and are placed under the same breeding environment as the subjects constituting the administration group and that constitute a control group to which the candidate substance for a drug has not been administered;
extracting a second feature amount from each of the plurality of second section images;
acquiring third feature amount information related to a third feature amount extracted from each of a plurality of third section images obtained by imaging tissue sections of a plurality of subjects constituting a control group to which the candidate substance was not administered in a past evaluation test; and
determining whether or not an abnormality is present in the tissue section included in the first section image on the basis of the first feature amount, the second feature amount, and the third feature amount information.
